# EUROPÄISCHE PATENTSCHRIFT

(11) **EP 1 178 781 B1**
(45) Veröffentlichungstag und Bekanntmachung des Hinweises auf die Patenterteilung: **28.09.2005**
(21) Anmeldenummer: 01913856.9
(22) Anmeldetag: 09.03.2001
(51) Int. Cl.: A61K 9/50

(54) **MEHRSCHICHTIGE ARZNEIFORM FÜR DIE COLONFREIGABE**
MULTILAYER PHARMACEUTICAL PRODUCT FOR RELEASE IN THE COLON
PRODUIT PHARMACEUTIQUE DE FORME GALENIQUE A PLUSIEURS COUCHES POUR LA LIBERATION DANS LE COLON

(30) Priorität: 17.03.2000 DE 10013029
(43) Veröffentlichungstag der Anmeldung: 13.02.2002
(73) Patentinhaber: Röhm GmbH & Co. KG, 64293 Darmstadt (DE)
(72) Erfinder: BECKERT, Thomas, 64297 Darmstadt (DE); PETEREIT, Hans-Ulrich, 64291 Darmstadt (DE); GUPTA, Vishal, K., Evanston, IL 60201 (US)
(86) Internationale Anmeldenummer: PCT/EP2001/002678
(87) Internationale Veröffentlichungsnummer: WO 2001/068058

(56) Entgegenhaltungen:
- WO-A-01/15667
- DE-A- 19 741 114
- US-A- 5 616 345
- V. K. GUPTA; ET AL.: "Statistical optimization of a novel multi-unit colonic delivery system containing multiple coatings of aqueous polymethacrylates" PROCEEDINGS 27TH INTERNATIONAL SYMPOSIUM ON CONTROLLED RELEASE OF BIOACTIVE MATERIALS, 7. Juli 2000 (2000-07-07), Seiten 453-454, XP002172814 paris (fr)
- PFORMULATE: "Excipients - Methacrylate-based coatings" HTTP://WWW.PFORMULATE.COM/METHACRYLATES.HT M, 2000, INTERNET

## Beschreibung

Die Erfindung betrifft eine mehrschichtige Arzneiform, aufgebaut aus einem Kern mit einem pharmazeutischen Wirkstoff, einem inneren Polymerüberzug und einem äußeren Polymerüberzug.

### Stand der Technik

(Meth)acrylat-Copolymere, die Monomere mit quaternären Ammoniumgruppen, z. B. Trimethylammoniumethlymethacrylat-Chlorid, enthalten und deren Verwendung für retardierende Arzneimittelüberzüge sind seit langem bekannt (z. B. aus EP-A 181 515 oder aus DE-PS 1 617 751). Die Verarbeitung erfolgt in organischer Lösung oder als wäßrige Dispersion z. B. durch Sprühen auf Arzneimittelkeme oder auch ohne Lösungsmittel in Gegenwart von Fließmitteln durch Aufbringen in der Schmelze (s. EP-A 0 727 205).

EP-A 629 398 beschreibt pharmazeutische Formutierungen, die einen Kern mit einem Wirkstoff und einer organischen Säure aufweisen, wobei der Kern zweischichtig umhüllt ist. Die innere Hülle wird dabei von einem retardierenden (Meth)acrylatcopolymer mit quaternären Ammoniumgruppen (EUDRAGIT® RS) gebildet, während die äußere Hülle einen magensaftresitenten Überzug aufweist, beispielweise ein Copolymer von Typ EUDRAGIT® L30D-55 (Ethylacrylat/Methacrylsäure, 50:50). Die erzielte Freisetzungscharakteristik kann durch eine zeitverzögerte, rasche Wirkstofffreissetzung bei erhöhten pH-Wert beschrieben werden.

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

EP 0 704 208 A2 beschreibt Überzugs- und Bindemittel für darmsaftlösliche Arzneiumhüllungen. Es handelt sich dabei um Copolymerisate aus 10 bis 25 Gew.-% Methacrylsäure, 40 bis 70 Gew.-% Methylacrylat und 20 bis 40 Gew-% Methylmethacrylat. Die Beschreibung erwähnt neben einschichtigen Überzügen auch mehrlagige Überzugssysteme. Diese können aus einem Kern, der z. B. einen basischen oder einen wasserempfindlichen Wirkstoff enthält, bestehen, weisen eine Isolierschicht aus einem anderen Überzugsmaterial, wie Celluloseether, Celluloseester oder einem kationischen Polymethacrylat z. B. von Typ EUDRAGIT®, u. a. auch EUDRAGIT® RS und RL, auf und werden dann zusätzlich mit der oben genannten darmsaftlöslichen Umhüllung versehen.

### Aufgabe und Lösung

Es sollte eine Arzneiform bereitgestellt werden, die im Magen nahezu keinen Wirkstoff abgibt und eine gleichmäßige und langanhaltende Wirkstoffabgabe im Darm, insbesondere kurz vor oder erst im Dickdarmbereich ermöglicht. Die Art der Wirkstoffabgabe soll insbesondere die Anforderung erfüllen, daß im Freisetzungstest nach USP zwei Stunden bei pH 1,2 und anschließendem Umpuffern auf pH 7,0 der enthaltene Wirkstoff im Zeitraum bis 2,0 Stunden nach Testbeginn zu weniger als 5 % und zum Zeitpunkt acht Stunden nach Testbeginn zu 30 bis 80 % freisetzt wird.

Die Aufgabe wird gelöst durch die Verwendung einer mehrschichtigen Arzneiform, die im wesentlichen aufgebaut ist aus
a) einem Kern mit einem pharmazeutischen Wirkstoff
b) einem inneren Überzug aus einem Copolymeren oder einer Mischung von Copolymeren, die sich aus 85 bis 98 Gew.-% radikalisch polymerisierten C1-bis C4-Alkylestern der Acryl- oder der Methacrylsäure und 15 bis 2 Gew.-% (Meth)acrylat-Monomeren mit einer quaternären Ammoniumgruppe im Alkylrest zusammensetzen und
c) einem äußeren Überzug aus einem Copolymeren, das sich aus 80 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt,

zur Herstellung einer Arzneiform, welche im Freisetzungstest nach USP zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80 % freisetzt.

Überraschenderweise wurde gefunden, daß sich das Wirkstofffreigabeprofil des inneren Überzugs nach Auflösung des äußeren, magensaftresistenten Überzugs vom einem Wirkstofffreigabeprofil unterscheidet, das erhalten wird, wenn man inneren Überzug ohne äußeren Überzug einsetzt. Man erhält bei Verwendung des aus EP 0 704 208 A2 bzw. EP-A 629 398 im Prinzip bekannten Aufbaus eine unerwartet langsame, sehr konstante Wirkstofffreisetzung.

Führt man den Freisetzungstest nach USP zwei Stunden bei pH 1,2 und anschließendem Umpuffern auf pH 7,5 durch, so sind zum Zeitpunkt sechs Stunden nach Testbeginn erst 30 bis 80 % des enthaltenen Wirkstoff freigesetzt. Dies ist insbesondere von Vorteil bei der Therapie von Erkrankungen, bei denen im Colon teilweise lokal erhöhte pH-Werte auftreten können, aber auch in diesen Fällen eine zu rasche Wirkstoffreisetzung vermieden werden soll, oder eine hinhaltende Wirktofffreigabe erreicht werden soll.

Die Wirkstofffreisetzung erfolgt zudem in unerwarteter Weise weitgehend unabhängig von der Dicke des äußeren Überzugs.

Offenbar kommt es während der Auflösung der äußeren Überzugsschicht zu einer Wechselwirkung mit der inneren Überzugsschicht. Das bisher nicht bekannte Freigabeprofil erhöht die Möglichkeiten des Fachmanns bei der Formulierung neuer Arzneiformen. Insbesondere ist die Freisetzungscharakteristik für einige Wirkstoffstoffe, die im Darm, insbesondere kurz vor oder erst im Dickdarmbereich möglichst konstant freigesetzt werden sollen, von Vorteil. Der offenbar nur äußerst geringe Einfluß der Dicke der äußeren Überzugsschicht auf das Freigabeprofil erhöht die Sicherheit in der Anwendung gegenüber Herstellungstoleranzen.

### Ausführung der Erfindung

Die Erfindung beschreibt die Verwendung einer mehrschichtigen Arzneiform, welche im Freisetzungstest nach USP für zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80 %, insbesondere zu 40 bis 70 % freisetzt.

Der Freisetzungstest nach USP (nach USP XXIV, Methode B, modifizierter Test für "enteric coated products") ist dem Fachmann bekannt. Die Versuchsbedingungen sind insbesondere: Paddle-Methode, 100 Umdrehungen pro Minute, 37 °C; pH 1,2 mit 0,1 N HCl, pH 7,0 durch Zugabe von 0,2 M Phosphatpuffer und Einstellen mit 2 N NaOH.

Die zu verwendende mehrschichtige Arzneiform besteht im wesentlichen aus einem Kern mit einem Wirkstoff, einem inneren und einem äußeren Überzug. In üblicher Weise können pharmazeutisch gebräuchliche Hilfsstoffe enthalten sein, die aber für die Erfindung nicht kritisch sind.

### Kern mit einem pharmazeutischen Wirkstoff

### Kerne

Träger bzw. Kerne für die Überzüge sind Tabletten, Granulate, Pellets, Kristalle von regelmäßiger oder unregelmäßiger Form. Die Größe von Granulaten, Pellets oder Kristallen liegt in der Regel zwischen 0,01 und 2,5 mm, die von Tabletten zwischen 2,5 und 30,0 mm. Die Träger enthalten üblicherweise zu 1 bis 95 % Wirkstoff sowie gegebenenfalls weitere pharmazeutische Hilfsstoffe. Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

Neben dem Wirkstoff können die Kerne weitere pharmazeutische Hilfsstoffe enthalten: Bindemittel, wie Lactose, Cellulose und deren Derivate, Polyvinylpyrrolidon (PVP), Feuchthaltemittel, Zerfallsförderer, Gleitmittel, Sprengmittel, Stärke und deren Derivate, Zucker Solubilisatoren oder andere.

Die Kerne können in üblicher Weise mit einem pharmazeutischen Wirkstoff versehen werden, indem man den entsprechenden Wirkstoff, z. B. als Wirkstoffpulver auf Trägerpartikel (Nonpareilles) mittels eines wäßrigen Bindemittels aufbringt. Die Wirkstoffkerne (Pellets) können nach Trocknung und Siebung in der gewünschten Größenfraktion erhalten werden (z. B. 0,7 bis 1 mm). Man bezeichnet dieses Verfahren u.a. als "Powder Layering".

### Pharmazeutische Wirkstoffe

Die im Sinne der Erfindung einsetzbaren pharmazeutische Wirkstoff sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen. Beispielhaft seien 5-Aminosalicylsäure, Corticosteroide (Budesonid), sowie Proteine (Insulin, Hormone, Antikörper) angeführt. Erfindungsgemäß können alle Wirkstoffe eingesetzt werden, die die gewünschte therapeutische Wirkung im Sinne der obigen Definition erfüllen und eine ausreichende Stabilität besitzen und deren Wirksamkeit gemäß der obigen Punkte über das Colon erreicht werden kann.

Wichtige Beispiele (Gruppen und Einzelsubstanzen) ohne Anspruch auf Vollständigkeit sind folgende:
Analgetika, Antibiotika, Antidiabetika, Antikörper
Chemotherapeutika,Corticoide/Corticosteroide
Entzündungshemmende Mittel, Enzympräparate
Hormone und deren Hemmstoffe, Nebenschilddrüsenhormone
Verdauungsfördernde Mittel, Vitamine, Zytostatika

Als Wirkstoffe sind insbesondere solche zu nennen, die im Darm, insbesondere kurz vor oder erst im Dickdarmbereich möglichst konstant freigesetzt werden sollen. Somit kann der pharmazeutische Wirkstoff ein Aminosalicylat, ein Sulfonamid oder ein Glucocorticoid sein, insbesondere 5-Aminosalicylsäure, Olsalazin, Sulfalazin, Prednison oder Budesonid.

### Beispiele für Wirkstoffe

Mesalazin
Sulfasalazin
Bethamethason-21-dihydrogenophosphat
Hydrocortison-21-acetat
Cromoglicinsäure
Dexamethason
Olsalazin-Na
Budesonid, Prednison
Bismunitrat, Karaya Gummi
Methylprednisolon-21-hydrogensuccinat
Myhrre, Kaffekohle, Kamillenblüttenextrakt
10% Suspension von Humanplacenta

### Neuere Wirkstoffe, bzw. Wirkstoffe in der Entwicklung und Prüfung (Literatur aus einschlägigen, dem Fachmann bekannten pharmazeutischen Datenbanken)

Balsalazid
Oral verabreichte Peptide (z.B. RDP 58)
Interleukin 6
Interleukin 12
Ilodecakin (Interleukin 10)
Nicotintartrat
5-ASA Konjugate (CPR 2015)
Monoclonaler Antikörper gegen Interleukin 12
Diethyldihydroxyhomospermin (DEHOHO)
Diethylhomospermin (DEHOP)
Cholecystokinin (CCK) Antagonist (CR 1795)
15 Aminosäure-Fragment eines 40 kd Peptids aus Magensaft (BPC 15)
Glucocorticoidanalogon (CBP 1011)
Natalizumab
Infliximab (REMICADE)
N-de-Acetyliertes Lysoglycosphingolipid (WILD 20)
Azelastine
Tranilast
Sudismase
Phosphorothioat Antisensoligonucleotid (ISIS 2302)
Tazofelone
Ropivacaine
5 Lipoxygenaseinhibitor (A 69412)
Sucralfat

### Innerer Überzug

Der innere Überzug besteht aus einem Copolymeren oder einer Mischung von Copolymeren, die sich aus 85 bis 98 Gew.-% radikalisch polymerisierten C1-bis C4-Alkylestern der Acryl- oder der Methacrylsäure und 15 bis 2 Gew.-% (Meth)acrylat-Monomeren mit einer quaternären Ammoniumgruppe im Alkylrest zusammensetzen.

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Bevorzugte C1- bis C4-Alkylestern der Acryl- oder der Methacrylsäure sind Methylacrylat, Ethylacrylat, Butylacrylat, Butylmethacrylat und Methylmethacrylat. Als (Meth)acrylat Monomer mit quaternären Ammoniumgruppen wird 2-Trimethylammoniumethylmethacrylat-Chlorid besonders bevorzugt.

Ein geeignetes Copolymer, kann z. B. aus aus 93 bis 98 Gew.-% radikalisch polymerisierten C1- bis C4-Alkylestern der Acryl- oder der Methacrylsäure und 7 - 2 Gew.-% 2-Trimethylammoniumethylmethacrylat-Chlorid hergestellt sein. Dabei können z. B. 50 - 70 Gew.-% Methylmethacrylat, 20 - 40 Gew.-% Ethylacrylat enthalten sein.

Ein entsprechendes Copolymer ist z. B. aus 65 Gew.-% Methylmethacrylat, 30 Gew.-% Ethylacrylat und 5 Gew.-% 2-Trimethylammoniumethylmethacrylat-Chlorid aufgebaut (EUDRAGIT® RS).

Ein weiteres geeignetes Copolymer, kann z. B. aus 85 bis weniger als 93 Gew.-% radikalisch polymerisierten C1- bis C4-Alkylestern der Acryl- oder der Methacrylsäure und zu 15 bis mehr als 7 Gew.-% 2-Trimethylammoniumethylmethacrylat-Chlorid hergestellt sein. Dabei können z. B. 50 - 70 Gew.-% Methylmethacrylat, 20-40 Gew.-% Ethylacrylat enthalten sein.

Geeignet ist ein Copolymer aus 60 Gew.-% Methylmethacrylat, 30 Gew.-% Ethylacrylat und 10 Gew.-% 2-Trimethylammoniumethlymethacrylat-Chlorid (EUDRAGIT® RL).

Der Mengenanteil des inneren Überzugs soll im Bereich von 2 bis 20 Gew.-% bezogen auf den Kern mit dem Wirkstoff betragen. Günstig ist die gleichzeitige Verwendung der beiden oben genannten Copolymer-Typen, bevorzugt derjenigen mit 5 und mit 10 Gew.-% 2-Trimethylammoniumethlymethacrylat-Chlorid (EUDRAGIT® RS und EUDRAGIT® RL) in Mischung. Das Mischungsverhältnis kann z. B. 20 : 1 bis 1 : 20, bevorzugt 10 : 1 bis 1 : 10 betragen

### Äußerer Überzug

Der äußere Überzug besteht aus einem Copolymeren, das sich aus 75 bis 95, insbesondere 85 bis 95 Gew.-% radikalisch polymerisierten C₁- bis C₄-Alkylestern der Acryl- oder der Methacrylsäure und 5 bis 25, bevorzugt 5 bis 15 Gew.-% (Meth)acrylat-Monomeren mit einer anionischen Gruppe im Alkylrest zusammensetzt.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein.

Besonders gui geeignet sind (Meth)acrylat Copolymere aus 10 bis 30 Gew.-%, Methylmethacrylat, 50 bis 70 Gew.-% Methylacrylat und 5 bis 15 Gew.-% Methacrylsäure (Typ EUDRAGIT® FS).

Der Mengenanteil des äußeren Überzugs soll im Bereich von 10 bis 50 Gew.-% bezogen auf das Gewicht des Kerns mit dem Wirkstoff und dem inneren Überzug betragen.

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden.

Dies kann durch einfaches Brechen extudierter und abgekühlter Granulatstränge oder Heißabschlag erfolgen.

Bevorzugt ist die Emulsionspolymerisation in wäßriger Phase in Gegenwart wasserlöslicher Initiatoren und (vorzugsweise anionischer) Emulgatoren (Siehe z. B. DE-C 2 135 073).

Das Emulsionpolymerisat wird vorzugsweise in Form einer 10- bis 50-gew.-prozentigen, insbesondere 30 bis 40-prozentigen wäßrigen Dispersion erzeugt und angewendet. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Verdickung der Überzugsmitteldispersion erwünscht sein sollte. Der Gewichtsmittelwert der Latex-Teilchengröße beträgt in der Regel 40 bis 100 nm, vorzugsweise 50 bis 70 nm, was eine verarbeitungstechnisch günstige Viskosität unter 1000 mPa - s gewährleistet.

Die Mindestfilmbildungstemperatur (MFT nach DIN 53 778) liegt für die meisten erfindungsgemäßen Überzugsmittel zwischen 0 und 25 °C, so daß die Verarbeitung bei Raumtemperatur ohne Weichmacherzusatz möglich ist. Die Reißdehnung der Filme, gemessen nach DIN 53 455, liegt bei einem Gehalt von höchstens 10 Gew.-% Triethylcitrat in der Regel bei 50 % oder mehr.

### Pharmazeutisch übliche Hilfsstoffe

Bei der Herstellung der mehrschichtigen Arzneiform können pharmazeutisch übliche Hilfsstoffe in üblicher Weise eingesetzt werden.

Trockenstellmittel (Antihaftmittel): Trockenstellmittel haben folgende Eigenschaften: sie verfügen über große spezifische Oberflächen, sind chemisch inert, sind gut rieselfähig und feinteilig. Aufgrund dieser Eigenschaften erniedrigen sie die Klebrigkeit von Polymeren, die als funktionelle Gruppen polare Comonomere enthalten.

### Beispiele für Trockenstellmittel sind:

Aluminiumoxid, Magnesiumoxid, Kaolin, Talkum, Kieselsäure (Aerosile), Bariumsulfat und Cellulose.

### Trennmittel

### Beispiele für Trennmittel sind:

Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc.. Übliche Mengenanteile liegen im Bereich von 0,05 Gew-% bis 5. bevorzugt 0,1 bis 3 Gew.-% bezogen auf das Copolymere.

Weitere pharmazeutisch übliche Hilfsstoffe: Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharmazeutisch übliche Hilfsstoffe können in Mengen von 0,001 Gew-% bis 30 Gew.-%, bevorzugt 0,1 bis 10 Gew.-% bezogen auf das Copolymere vorliegen.

Weichmacher: Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phthalsäurealkylester, Sebacinsäurealkylester, Sucroseester, Sorbitanester, Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 1 und 35, bevorzugt 2 bis 10 Gew.-% .-%, bezogen auf das (Meth)acrylatCopolymere.

### Applikationsformen

Die beschriebene Arzneiform kann als überzogene Tablette, in Form einer Tablette aus verpreßten Pellets oder in Form von Pellets vorliegen, die in eine Kapsel, z. B. aus Gelatine, Stärke oder Cellulosederivaten, eingefüllt sind.

### BEISPIELE

### Beispiel 1 a bis 1 d

Herstellung von mehrschichtigen Arzneiformen, bestehend aus einem Kern mit 5-Aminosalicylsäure als Wirkstoff, einem inneren Überzug aus einer Mischung von EUDRAGIT® RS und RL im Verhältnis 8 : 2 und einem äußeren Überzug aus EUDRAGIT® FS in Schichtdicken von 15, 20,25 bzw. 30 Gew.-%.

### Eingesetzte Copolymere/Polymerdispersionen:

EUDRAGIT® RS 30 D: 30 %-ige wäßrige Dispersion, enthaltend ein Copolymer aus 65 Gew.-% Methylmethacrylat, 30 Gew.-% Ethylacrylat und 5 Gew.-% 2-Trimethylammoniumethylmethacrylat-Chlorid.
EUDRAGIT® RL 30 D: 30 %-ige wäßrige Dispersion, enthaltend ein Copolymer aus 60 Gew.-% Methylmethacrylat, 30 Gew.-% Ethylacrylat und 10 Gew.-% 2-Trimethylammoniumethylmethacrylat-Chlorid..
EUDRAGIT® FS 30D: 30%-ige Dispersion, enthaltend ein Copolymer aus 25 Gew.-%, Methylmethacrylat, 65 Gew.-% Methylacrylat und 10 Gew.-% Methacrylsäure.

Wirkstoffhaltige Kerne (Pellets) wurden im "Powder-Layering-Verfahren" hergestellt. Dazu wurden in Gew.-% eingesetzt:

| Als Kerne | |
|---|---|
| Nonpareils (0,5 - 0,6 mm) | 40 g |

| Als "Layering Powder" | |
|---|---|
| 5-Aminosalicylsäure | 48,75 g |
| Lactose D80 | 10,65 g |
| Aerosil 200 | 0,6 g |
| Summe: | 100 g |

| Als Binder | |
|---|---|
| Kollidon 25 | 5 g |
| Wasser | 95 g |
| Summe: | 100 g |

Die Kerne wurden in einem Wirbelschichtgerät mit dem Binder besprüht und das "Layering-Powder" jeweils in kleinen Portionen zugegeben. Versuchsbedingungen für das Powder-Layering im Einzelnen:

| | |
|---|---|
| Ansatzgröße (Batch/Nonpareils) | 800 g |
| Zielgröße Wirkstoff/Kern | 975 g |
| Sprühpistolentyp | Walter "Bingo" |
| Sprühdüsendurchmesser | 1,0 mm |
| Pan Speed | 40 Upm |
| Sprühwinkel (Inclination angle pan) | 30° |
| Abstand Wirbelbett zu Sprühpistole | 10 cm |
| Sprühdruck | 0,4 bar |
| Auftragsmenge Binder (trocken) | 13,75 g |
| Sprühzeit Binder | 70 min |
| Sprührate Binder | 0,2 - 0,25 g / min |
| Zeit (Layering) | 66 min |
| Menge pro Schicht | 18g |
| Trocknungsdauer im Wirbelbett nach Auftrag | 5 min |
| Trocknung | 24 h bei 40 °C |
| Rechnerische Ausbeute | 95 - 98 % |

Die so erhaltenen Pellets wurden für 24 Stunden bei 40 °C getrocknet. Anschließend wurden Pellets der Größenfraktion von 0,7 bis 1,0 mm ausgesiebt und für die Überzugsverfahren im Sprühauftrag eingesetzt.

Versuchsbedingungen für den Auftrag des inneren und des äußeren Copolymerüberzugs im Einzelnen:

| | |
|---|---|
| Ansatzgröße (Batch) | 800 g |
| Sprühdüsendurchmesser | 1,2 mm |
| Abstand Wirbelbett zu Sprühpistole | nahe |
| Sprühdruck | 2 bar |
| Eingeblasene Luftmenge | 85 - 95 m³/h |
| dabei Lufttemperatur | 30 - 40 °C |
| Ablufttemperatur | 26 - 30 °C |
| Temperatur im Wirbelbett | 23 - 26 °C |
| Sprührate | 10 g / min |
| Trocknungsdauer im Wirbelbett nach Auftrag | 5 min |
| Trocknung | 24 h bei 40 °C |
| Rechnerische Ausbeute | 96 - 98 % |

### Innere Überzugsschicht (Sprühauftrag).

Zum Auftrag des inneren Überzugs wurde folgende Mischung eingesetzt:

| | |
|---|---|
| EUDRAGIT® RS 30 D | 173 g |
| EUDRAGIT® RL 30 D | 43 g |
| Glycerolmonostearat | 3 g |
| Triethylcitrat | 13 g |
| Tween 80 (33 % aq.) | 3 g |
| Wasser | 173 g |

Dies ergibt einen Feststoffgehalt der Sprühsuspension von 20 %. Insgesamt wurden 10,1 % Feststoff, entsprechend 8 Gew.-% Polymer auf die wrkstoffhaltigen Kerne aufgetragen.

### Äußerer Überzug (Sprühauftrag)

| | |
|---|---|
| EUDRAGIT® FS 30 D | 800 g |
| Glycerolmonostearat | 12 g |
| Tween 80 (33% aq.) | 15 g |
| Wasser | 458 g |

Dies ergibt einen Feststoffgehalt der Sprühsuspension von 20 %. Insgesamt wurden 32,1 % Feststoff, entsprechend 30 Gew.-% Polymer auf die wrkstoffhaltigen, mit dem inneren Überzug versehenen Kerne aufgetragen. Zu verschiedenen Zeitpunkten wurden Kerne mit entsprechend 15, 20, 25 und zuletzt 30 Gew.-% Polymerüberzug entnommen (Beispiele 1a, 1b, 1 c bzw. 1 d).

### Beispiel 2

Herstellung von mehrschichtigen Arzneiformen, bestehend aus einem Kern mit 5-Aminosalicylsäure als Wirkstoff aus Beispiel 1, einem inneren Überzug aus einer Mischung von EUDRAGIT® RS und RL im Verhältnis 6,8 : 3,2 in Schichtdicke von 6,8 Gew.-% und einem äußeren Überzug aus EUDRAGIT® FS in einer Schichtdicke von 14 Gew.-%.

Versuchsbedingungen für den Auftrag des inneren und des äußeren Copolymerüberzugs im Einzelnen:

| | |
|---|---|
| Ansatzgröße (Batch) | 800 g |
| Sprühdüsendurchmesser | 1,2 mm |
| Abstand Wirbelbett zu Sprühpistole | nahe |
| Sprühdruck | 2 bar |
| Eingeblasene Luftmenge | 65 - 85 m³/h |
| dabei Lufttemperatur | 30 - 40 °C |
| Ablufttemperatur | 26 - 30 °C |
| Temperatur im Wirbelbett | 23 - 27 °C |
| Sprührate | 10 g / min |
| Trocknungsdauer im Wirbelbett nach Auftrag | 5 min |
| Trocknung | 24 h bei 40 °C |
| Rechnerische Ausbeute | 95 - 98 % |

### Innere Überzugsschicht (Sprühauftrag).

Zum Auftrag des inneren Überzugs wurde folgende Mischung eingesetzt:

| | |
|---|---|
| EUDRAGIT® RS 30 D | 123 g |
| EUDRAGIT® RL 30 D | 58 g |
| Glycerolmonostearat | 1,5 g |
| Triethylcitrat | 11 g |
| Tween 80 (33 % aq.) | 1,5 g |
| Wasser | 147 g |

Dies ergibt einen Feststoffgehalt der Sprühsuspension von 20 %. Insgesamt wurden 8,6 % Feststoff, entsprechend 6,8 Gew.-% Polymer auf die wirkstoffhaltigen Kerne aufgetragen.

### Äußerer Überzug (Sprühauftrag)

| | |
|---|---|
| EUDRAGIT® FS 30 D | 370 g |
| Glycerolmonostearat | 5,5 g |
| Tween 80 (33% aq.) | 2,75 g |
| Wasser | 218 g |

Dies ergibt einen Feststoffgehalt der Sprühsuspension von 20 %. Insgesamt wurden 14,9 % Feststoff, entsprechend 14 Gew.-% Polymer auf die wrkstoffhaltigen, mit dem inneren Überzug versehenen Kerne aufgetragen. (Beispiel 2).

### Beispiel 3

Herstellung von mehrschichtigen Arzneiformen, bestehend aus einem Kern mit 5-Aminosalicylsäure als Wirkstoff aus Beispiel 1, einem inneren Überzug aus einer Mischung von EUDRAGIT® RS und RL im Verhältnis 8 : 2 in Schichtdicke von 5 Gew.-% und einem äußeren Überzug aus EUDRAGIT® FS in einer Schichtdicke von 20 Gew.-%.

Versuchsbedingungen für den Auftrag des inneren und des äußeren Copolymerüberzugs im Einzelnen:

| | |
|---|---|
| Ansatzgröße (Batch) | 800 g |
| Sprühdüsendurchmesser | 1,2 mm |
| Abstand Wirbelbett zu Sprühpistole | nahe |
| Sprühdruck | 2 bar |
| Eingeblasene Luftmenge | 65 - 85 m³/h |
| dabei Lufttemperatur | 30 - 40 °C |
| Ablufttemperatur | 26 - 30 °C |
| Temperatur im Wirbelbett | 23 - 27 °C |
| Sprührate | 10 g / min |
| Trocknungsdauer im Wirbelbett nach Auftrag | 5 min |
| Trocknung | 24 h bei 40 °C |
| Rechnerische Ausbeute | 95 - 98 % |

### Innere Überzugsschicht (Sprühauftrag).

Zum Auftrag des inneren Überzugs wurde folgende Mischung eingesetzt:

| | |
|---|---|
| EUDRAGIT® RS 30 D | 106 g |
| EUDRAGIT® RL 30 D | 27 g |
| Glycerolmonostearat | 2 g |
| Triethylcitrat | 8 g |
| Tween 80 (33 % aq.) | 2 g |
| Wasser | 115 g |

Dies ergibt einen Feststoffgehalt der Sprühsuspension von 20 %. Insgesamt wurden 6,5 % Feststoff, entsprechend 5 Gew.-% Polymer auf die wirkstoffhaltigen Kerne aufgetragen.

### Äußerer Überzug (Sprühauftrag)

| | |
|---|---|
| EUDRAGIT® FS 30 D | 533 g |
| Glycerolmonostearat | 8 g |
| Tween 80 (33% aq.) | 4 g |
| Wasser | 315 g |

Dies ergibt einen Feststoffgehalt der Sprühsuspension von 20 %. Insgesamt wurden 21,5 % Feststoff, entsprechend 20 Gew.-% Polymer auf die wirkstoffhaltigen, mit dem inneren Überzug versehenen Kerne aufgetragen (Beispiel 3).

### Beispiel 4: Versuche zur Wirkstofffreigabe

### Methode:

Nach USP XXIV, Methode B, modifizierter Test für "enteric coated products. Die Versuchsbedingungen sind insbesondere: Paddle-Methode, 100 Umdrehungen pro Minute, 37 °C; pH 1,2 mit 0,1 N HCl, pH 7,0 durch Zugabe von 0,2 M Phosphatpuffer und Einstellen mit 2 N NaOH.

Es wurden jeweils Pellets aus den Beispielen 1 a bis 1d, sowie nicht überzogene Pellets und nur mit dem inneren Überzug versehene Pellets als Vergleich eingesetzt.

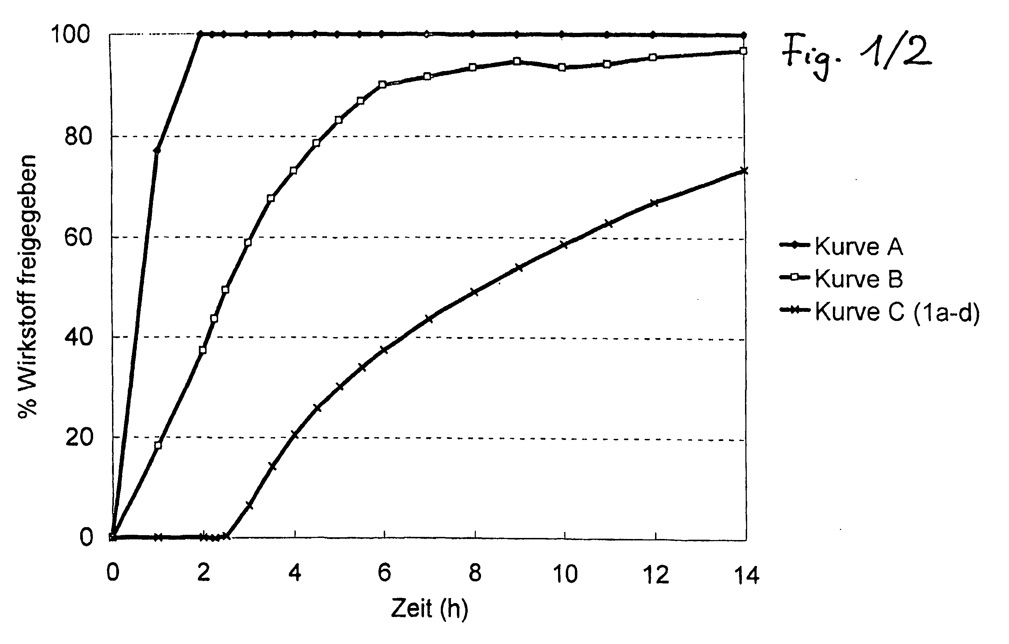

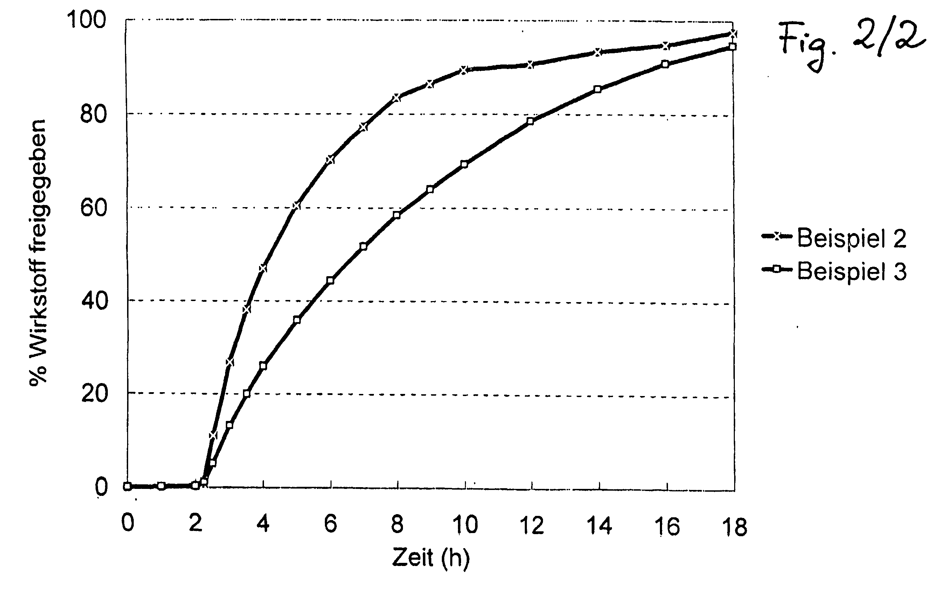

200 mg Pellets wurden in der Versuchsapparatur (DT 80, Erweka, Switzerland) zu 700 ml 0,1 N HCl gegeben. Nach 2 Stunden wurde mit 200 ml 0,2 M Phosphatpuffer umgepuffert und mittels 2N HCl bzw. 2N NaOH auf pH 7,0 (Beispiel 1a-d, Fig 1/2) oder pH 7,5 (Beispiele 2 und 3, Fig 2/2) eingestellt. Die Wirkstofffreigabe wurde UV-spektrometrisch verfolgt.

**Fig 1/2** zeigt die erhaltenen Wirkstofffreigabeprofile bei pH 7,0
Kurve A: nicht überzogene Pellets
Kurve B: nur mit dem inneren Überzug versehene Pellets
Kurve C: entsprechend den Beispielen 1a bis 1 d überzogene Pellets.
Da die Freigabeprofile 1a bis 1 d praktisch identisch waren, sind sie in nur einer Kurve zusammengefaßt.

Ergebnis (Fig. 1/2): Die in der Kurve C zusammengefassten Freigabeprofile verlaufen nach Auflösung der äußeren Überzugsschicht deutlich flacher als die Kurve B. Das Freigabeprofil der Kurve C ist verläuft dabei praktisch unabhängig von der Dicke der äußeren Überzugsschicht der geprüften Pellets 1a bis 1d.

**Fig. 2/2** zeigt die erhaltenen Wirkstofffreigabeprofile bei pH 7,5: Beispiel 2: Innerer Überzug aus einer Mischung von EUDRAGIT® RS und RL im Verhältnis 6,8 : 3,2 in Schichtdicke von 6,8 Gew.-% und einem äußeren Überzug aus EUDRAGIT® FS in einer Schichtdicke von 14 Gew.-%.

Beispiel 3: Innerer Überzug aus einer Mischung von EUDRAGIT® RS und RL im Verhältnis 8 : 2 in Schichtdicke von 5 Gew.-% und einem äußeren Überzug aus EUDRAGIT® FS in einer Schichtdicke von 20 Gew.-%.

Ergebnis (Fig. 2/2): Selbst bei auf pH 7,5 erhöhtem pH-Wert erfolgt eine retardierende Wirkstofffreigabe.

## Patentansprüche

1. Verwendung einer mehrschichtigen Arzneiform, die im wesentlichen aufgebaut ist aus
a) einem Kern mit einem pharmazeutischen Wirkstoff
b) einem inneren Überzug aus einem Copolymeren oder einer Mischung von Copolymeren, die sich aus 85 bis 98 Gew.-% radikalisch polymerisierten C1-bis C4-Alkylestern der Acryl- oder der Methacrylsäure und 15 bis 2 Gew.-% (Meth)acrylat-Monomeren mit einer quaternären Ammoniumgruppe im Alkylrest zusammensetzen und einem
c) einem äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt
zur Herstellung einer Arzneiform, welche im Freisetzungstest nach USP zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80 % freisetzt.

2. Verwendung nach Anspruch 1, **dadurch gekennzeichnet, daß** der pharmazeutische Wirkstoff ein Aminosalicylat, ein Sulfonamid oder ein Glucocorticoid ist.

3. Verwendung nach Anspruch 2, **dadurch gekennzeichnet, daß** der pharmazeutische Wirkstoff 5-Aminosalicylsäure, Olsalazin, Sulfalazin, Prednison oder Budesonid ist.

4. Verwendung nach einem oder mehreren der Ansprüche 1 bis 3, **dadurch gekennzeichnet, daß** die Schichtdicke des inneren Überzuges 2 bis 50% Gew.-% des Kerns mit einem pharmazeutischen Wirkstoff ausmacht.

5. Verwendung nach einem oder mehreren der Ansprüche 1 bis 4, **dadurch gekennzeichnet, daß** die Schichtdicke des äußeren Überzugs 5 bis 50 Gew.-% bezogen auf das Gewicht des Kerns mit dem pharmazeutischen Wirkstoff und dem inneren Überzug ausmacht.

6. Verwendung nach einem oder mehreren der Ansprüche 1 bis 5, **dadurch gekennzeichnet, daß** der innere Überzug eine Mischung aus einem Copolymer aus 93 bis 98 Gew.-% radikalisch polymerisierten C1- bis C4-Alkylestern der Acryl- oder der Methacrylsäure und 7 - 2 Gew.-% 2-Trimethylammoniumethylmethacrylat-Chlorid und einem Copolymer aus 85 bis weniger als 93 Gew.-% radikalisch polymerisierten C1- bis C4-Alkylestern der Acryl- oder der Methacrylsäure und zu 15 bis mehr als 7 Gew.-% 2-Trimethylammoniumethylmethacrylat-Chlorid ist.

7. Verwendung nach Anspruch 6, **dadurch gekennzeichnet, daß** das Mischungverhältnis 20 : 1 bis 1 : 20 beträgt.

8. Verwendung nach einem oder mehreren der Ansprüche 1 bis 7, **dadurch gekennzeichnet, daß** die Arzneiform in Form von Tabletten, Pellets, aus Pellets verpreßten Tabletten oder in Kapseln verfüllter Pellets vorliegt.

## Claims

1. Use of a multi-layered pharmaceutical preparation essentially made up of
a) a core containing a pharmaceutical active substance
b) an inner coating comprising a copolymer or a mixture of copolymers which are composed of 85 to 98 wt.% of radically polymerised C₁ - C₄-alkyl esters of acrylic or methacrylic acid and 15 to 2 wt.% of (meth) acrylate monomer(s) with a quaternary ammonium group in the alkyl radical and
c) an outer coating comprising a copolymer which is composed of 75 to 95 wt.% of radically polymerised C₁ - C₄-alkyl esters of acrylic or methacrylic acid and 5 to 25 wt.% of (meth)acrylate monomer(s) with an anionic group in the alkyl radical,
for the production of a pharmaceutical preparation which in the USP release test, with two hours at pH 1.2 followed by buffering to pH 7.0, releases less than 5 % of the active substance contained therein within a period of up to 2.0 hours after the start of the test and 30 to 80 % of the substance by a time eight hours after the start of the test.

2. Use according to claim 1, **characterised in that** the pharmaceutical active substance is an aminosalicylate, a sulphonamide or a glucocorticoid.

3. Use according to claim 2, **characterised in that** the pharmaceutical active substance is 5-aminosalicylic acid, olsalazine, sulphalazine, prednisone or budesonide.

4. Use according to one or more of claims 1 to 3, **characterised in that** the layer thickness of the inner coating makes up 2 to 50 wt.% of the core containing a pharmaceutical active substance.

5. Use according to one or more of claims 1 to 4, **characterised in that** the layer thickness of the outer coating makes up 5 to 50 wt.% based on the weight of the core containing the pharmaceutical active substance and the inner coating.

6. Use according to one or more of claims 1 to 5, **characterised in that** the inner coating is a mixture of a copolymer of 93 to 98 wt.% of radically polymerised C₁ - C₄-alkyl esters of acrylic or methacrylic acid and 7 to 2 wt.% of 2-trimethylammoniumethyl methacrylate chloride and a copolymer of 85 to less than 93 wt.% of radically polymerised C₁ - C₄-alkyl esters of acrylic or methacrylic acid and up to 15 to more than 7 wt.% of 2-trimethylammoniumethyl methacrylate chloride.

7. Use according to claim 6, **characterised in that** the mixing ratio is 20 : 1 to 1 : 20.

8. Use according to one or more of claims 1 to 7, **characterised in that** the pharmaceutical preparation is in the form of tablets, pellets, tablets compressed from pellets, or pellets packed into capsules.

## Revendications

1. Utilisation d'une forme médicamenteuse à plusieurs couches, et constituée pour l'essentiel de
a) un noyau comportant une substance active pharmaceutique,
b) un revêtement interne fait d'un copolymère ou d'un mélange de copolymères, qui se compose de 85 à 98 % en poids d'esters alkyliques en C₁ à C₄ de l'acide acrylique ou méthacrylique polymérisables par voie radicalaire, et de 15 à 2 % en poids de monomères de (méth)acrylates comportant un groupe ammonium quaternaire dans le radical alkyle, et
c) un revêtement externe fait d'un copolymère qui se compose de 75 à 95 % en poids d'esters alkyliques en C₁ à C₄ de l'acide acrylique ou de l'acide méthacrylique polymérisables par voie radicalaire, et de 5 % à 25 % en poids de monomères de (méth)acrylates ayant un groupe anionique dans le radical alkyle,
pour la production d'une forme médicamenteuse qui, dans le test de libération selon la pharmacopée américaine pendant deux heures à pH 1,2, puis changement de tampon à pH 7,0, libère la substance active qui y est contenue, à moins de 5 % dans un délai allant jusqu'à 2,0 heures après le début du test, et 30 à 80 % huit heures après le début du test.

2. Utilisation selon la revendication 1,
**caractérisée en ce que**
la substance active pharmaceutique est un aminosalicylate, un sulfonamide ou un glucocorticoïde.

3. Utilisation selon la revendication 2,
**caractérisée en ce que**
la substance active pharmaceutique est l'acide 5-aminosalicylique, l'olsalazine, la sulfalazine, la prednisone ou le budesonide.

4. Utilisation selon une ou plusieurs des revendications 1 à 3,
**caractérisée en ce que**
l'épaisseur de couche du revêtement interne constitue de 2 à 50 % en poids du noyau avec une substance active pharmaceutique.

5. Utilisation selon une ou plusieurs des revendications 1 à 4,
**caractérisée en ce que**
l'épaisseur de couche du revêtement externe constitue de 5 à 50 % en poids par rapport au poids du noyau avec la substance active pharmaceutique et le revêtement interne.

6. Utilisation selon une ou plusieurs des revendications 1 à 5,
**caractérisée en ce que**
le revêtement interne est un mélange d'un copolymère constitué de 93 à 98 % en poids d'esters alkyliques en C₁ à C₄, polymérisés par voie radicalaire, de l'acide acrylique ou méthacrylique, et de 7 à 2 % en poids de chlorure de méthylméthacrylate de 2-triméthylammonium et d'un copolymère constitué de 85 à moins de 93 % en poids d'esters alkyliques en C₁ à C₄, polymérisés par voie radicalaire, de l'acide acrylique ou méthacrylique, et de 15 à plus de 7 % en poids de chlorure de méthylméthacrylate de 2-triméthylammonium.

7. Utilisation selon la revendication 6,
**caractérisée en ce que**
le rapport de mélange est de 20 : 1 à 1 : 20.

8. Utilisation selon une ou plusieurs des revendications 1 à 7,
**caractérisée en ce que**
la forme médicamenteuse se présente sous forme de comprimés, de boulettes, de comprimés produits à partir de boulettes ou de boulettes versées dans des capsules.
